Europäisches Patentamt

European Patent Office

Office européen des brevets

(19)

(11) Veröffentlichungsnummer: **0 374 415**
**A2**

(12) **EUROPÄISCHE PATENTANMELDUNG**

(21) Anmeldenummer: 89119449.0

(22) Anmeldetag: 20.10.89

(51) Int. Cl.5: **C11D 3/37, C11D 17/00,**
**C11D 1/74**

(30) Priorität: 20.12.88 DE 3842823

(43) Veröffentlichungstag der Anmeldung:
27.06.90 Patentblatt 90/26

(84) Benannte Vertragsstaaten:
AT BE CH DE ES FR GB GR IT LI LU NL SE

(71) Anmelder: HÜLS AKTIENGESELLSCHAFT
Patentabteilung / PB 15 - Postfach 13 20
D-4370 Marl 1(DE)

(72) Erfinder: Ruback, Wulf, Dr.
Moltkestrasse 15 a
D-4350 Recklinghausen(DE)

(54) **Verdickungsmittel für wässrige Tensidformulierungen.**

(57) Die für optimale anwendungstechnische Eigenschaften bekannten und notwendigen Verdickungsmittel für wäßrige Tensidformulierungen weisen Nachteile im Hinblick auf Herstellung und Wirksamkeit auf. So sind z. B. Polyetherester nur unter vermindertem Druck, hoher Temperatur und langer Reaktionszeit herstellbar. Elektrolyte z. B. sind nur in sehr hohen Einsatzkonzentrationen verdickend wirksam.

Die neuen Verdickungsmittel sollen diese Nachteile nicht aufweisen.

Es wird hierzu die Verwendung als Verdickungsmittel von Umsetzungsprodukten aus der Reaktion eines Polyanhydrids mit mindestens 2 Anhydridfunktionen mit Polyglykolethern der Formel $R-O(CHR_1-CHR_2-O)_x H$ vorgeschlagen.

Diese Verdickungsmittel wirken schon bei Einsatzkonzentrationen von 1 bis 5 % verdickend und sind bei milden Bedingungen herstellbar.

Verwendung von Umsetzungsprodukten aus der Reaktion eines Polyanhydrids mit Polyglykolethern als Verdickungsmittel in wäßrigen Tensidlösungen.

EP 0 374 415 A2

## Verdickungsmittel für wäßrige Tensidformulierungen

Für optimale anwendungstechnische Eigenschaften wäßriger Lösungen, Emulsionen oder Suspensionen von Tensiden ist deren Viskosität von besonderer Bedeutung. Aus diesem Grunde werden solche Mischungen oft durch Verdickungsmittel auf höhere Viskositäten eingestellt. Dadurch wird die Handhabung zum Beispiel eines Haarshampoos erleichtert. Es läßt sich einfach dosieren und leichter anwenden, da es sich nur langsam auf der Hand verteilt und nicht von den Haaren fließt. Darüber hinaus bewirkt eine erhöhte Viskosität eine verbesserte Lagerstabilität von Formulierungen, die unlösliche Bestandteile, wie z. B. Perlglanzpigmente oder Antischuppenmittel, enthalten.

Es sind eine Vielzahl von Verdickungsmitteln bekannt, mit denen sich die Viskosität wäßriger Tensidformulierungen erhöhen läßt.

So werden Elektrolyte, wie z. B. Kochsalz, zur Verdickung von Alkylethersulfaten eingesetzt.

Allgemein als Verdickungsmittel anwendbar sind Celluloseether, Fettsäurealkanolamide, N-Alkyl-Fettsäureglucamide, Acetamidocarboxylate (US 4 704 226), Polyethylenglykoldistearat, Propandiolpolyoxyethylenetherdifettsäureester (GB 2 129 004), Alkylpolyethylenglykolether-Fettsäureester (DE-OS 35 41 813), Polyethylenglykoldialkylether (DE-OS 35 51 535), oder auch Polyester aus Polyalkylenoxid und dimerisierten Fettsäuren (DE-OS 36 00 263).

Diese Verdickungsmittel weisen aber Nachteile im Hinblick auf ihre Herstellung und Wirksamkeit auf. So können beispielsweise einerseits die Polyetherester nur aufwendig unter vermindertem Druck bei hohen Temperaturen hergestellt werden. Dabei sind zusätzlich oft noch lange Reaktionszeiten nötig, was sehr leicht zu einer Farbverschlechterung der Produkte führt.

Auf der anderen Seite sind Elektrolyte nur in Formulierungen mit sehr hohem Ethersulfatanteil einsetzbar; Celluloseether lassen sich nur schwierig einarbeiten, neigen zum "Faden ziehen" und sind mikrobiell sehr anfällig; die Fettsäureamide sind erst bei höheren Einsatzkonzentrationen wirksam.

Dieser Erfindung liegt daher die Aufgabe zugrunde, hochwirksame Verdickungsmittel aufzufinden, die zudem bei milden Reaktionsbedingungen schnell herzustellen sind.

Diese Aufgabe wurde erfindungsgemäß dadurch gelöst, daß man Verdickungsmittel verwendet, die aus der Reaktion eines Polyanhydrids mit mindestens 2 Anhydridfunktionen mit Polyglykolethern der allgemeinen Formel $R-O(CHR_1-CHR_2-O)_x H$ gewonnen werden, und diese Verdickungsmittel in einer Konzentration von 0,1 bis 10 %, bevorzugt 1 bis 5 %, bezogen auf wäßrige Tensidlösung der Tensidformulierung, zusetzt.

Der Gegenstand der Erfindung ist daher die Verwendung von Umsetzungsprodukten von Polyanhydriden, welche mindestens zwei Anhydridfunktionen pro Molekül aufweisen, mit Polyglykolethern der Formel $R-O(CHR_1-CHR_2-O)_x H$ als Verdickungsmittel für wäßrige Tensidformulierungen, wobei R für gesättigte oder ungesättigte Alkylreste mit 8 bis 24 C-Atomen oder für Alkylarylreste, $R_1$ und $R_2$ für H, $CH_3$, $C_2H_5$ mit der Maßgabe, daß mindestens ein Rest H ist, und x für ganze Zahlen von 20 bis 150 stehen und daß das Verhältnis von Polyglykolether zu Anhydridfunktion 0,5 : 1 bis 2 : 1 ist.

Und zwar setzt man Polyanhydride wie Pyromellithsäuredianhydrid, meso-Butan-1,2,3,4-tetracarbonsäuredianhydrid, all-cis-Cyclopentan-1,2,3,4-tetracarbonsäuredianhydrid, 3,3´,4,4´-Benzophenontetracarbonsäuredianhydrid oder auch Maleinsäureanhydridoligomere mit Fettalkoholethoxilaten ($C_8$ bis $C_{24}$, bevorzugt $C_{14}$ bis $C_{20}$, gesättigt oder ungesättigt, 20 bis 150 mol EO, bevorzugt 30 bis 100 mol EO, pro mol Fettalkohol) gegebenenfalls in Gegenwart geringer Mengen eines Veresterungskatalysators, bevorzugt p-Toluolsulfonsäure oder 1-Methylimidazol, um. Das Verhältnis von Polyglykolether zu Anhydridfunktion liegt bei 0,5 : 1 bis 2 : 1, bevorzugt bei 0,8 : 1 bis 1 : 3,1. Die Reaktion läuft bei sehr milden Bedingungen (Normaldruck; 80 bis 140 °C, vorzugsweise 100 bis 120 °C) ab und ist in der Regel nach 1 bis 5 Stunden beendet. Zweckmäßigerweise läßt man die Reaktion in einer Stickstoffatmosphäre ablaufen. Man erhält farblose bis schwach gelbliche Produkte.

Durch die Kopplung über das Di(poly)anhydrid bildet sich eine lange, hydrophile Polyetherkette mit hydrophoben Endgruppen. Die nach der Reaktion an dem verbindenden, an sich hydrophoben, Kohlenstoffgerüst verbleibenden freien Carboxylgruppen solubilisieren dieses so gut, daß man eine durchgehende hydrophile Kette erhält.

Mit den auf die oben beschriebene Art und Weise erhaltenen Produkten lassen sich klare, hochviskose Einstellungen der gängigen nichtionischen und/oder anionischen und/oder amphoteren Tenside in einfacher Weise herstellen. Diese können pharmazeutische, kosmetische, Haushalts- oder auch technische Präparate sein. Dabei ist auch die Kombination der erfindungsgemäßen Produkte mit anderen üblichen Bestandteilen dieser Präparate möglich. So können sie beispielsweise in Haarpflegemittel, Hautreinigungsmittel, Haarverformungsmittel, Salbe, Geschirrspülmittel, Waschmittel, Kraftfahrzeugreinigungsmittel, Haushaltsreinigungsmittel und andere technische Reinigungspärparate eingearbeitet werden.

Der mengenmäßige Anteil der beschriebenen Produkte in den verschiedensten tensidhaltigen Präparaten kann je nach der gewünschten Viskosität zwischen 0,1 bis etwa 10 %, vorzugsweise jedoch zwischen 1 und 5 %, bezogen auf das Gewicht der Fertigformulierung, schwanken. Die Einarbeitung dieser Produkte in die zu verdickenden Lösungen, Suspensionen oder Emulsionen erfolgt in an sich bekannter Weise durch Auflösen des Verdickungsmittels, evtl. unter Erwärmen in Wasser bzw. in einer wasserhaltigen Phase.

In einigen anionischen Tensidsystemen wie z. B. alkylethersulfathaltigen Formulierungen beobachtet man dabei überraschenderweise einen ausgesprochenen Synergismus mit Elektrolyten wie z. B. Kochsalz, wodurch die Gesamtmenge des zur Einstellung einer bestimmten Viskosität benötigten Viskositätsreglers reduziert wird, bzw. die an sich schon überraschend guten Verdickungseigenschaften der erfindungsgemäßen Produkte dadurch noch verstärkt werden.

Zusätzlich zu den rheologischen Eigenschaften verbessern die erfindungsgemäßen Viskositätsregler die Schäumeigenschaften der sie enthaltenden Formulierungen. Bei entsprechenden Haarpflegepräparaten beobachtet man einen Konditioniereffekt, d. h. die Kämmbarkeit und Fülle des Haares wird verbessert.

Durch die nachfolgenden Beispiele wird die Erfindung näher erläutert.

### Herstellbeispiel 1:

In einem Dreihalskolben werden 250 g (= 53,6 mmol) eines $C_{16}$- bis $C_{18}$-Fettalkoholpolyglykolethers (100 mol Ethylenoxid pro Mol Fettalkohol) in einer Stickstoffatmosphäre geschmolzen und auf 80 °C erwärmt. Bei dieser Temperatur werden 5,85 g (= 26,8 mmol) Pyromellithsäureanhydrid zugegeben und weiter erwärmt. Nach Erreichen von 100 °C werden 1,28 g (= 0,5 Gew.-%) N-Methyl-imidazol zugesetzt und weitere 3 Stunden bei dieser Temperatur gerührt. Danach wird das Produkt abgelassen, abgekühlt und für die weiteren Versuche verwendet.

Die Säurezahl des Produktes wird mit 12,98 mg KOH/g bestimmt.

### Herstellbeispiel 2:

In einem Dreihalskolben werden in einer Stickstoffatmosphäre 100 g (= 41,6 mmol) eines $C_{16}$- bis $C_{18}$-Fettalkoholpolyglykolethers (50 mol Ethylenoxid pro mol Fettalkohol) geschmolzen und bei 80 °C mit 4,12 g (= 20,8 mmol) meso-Butan-1,2,3,4-tetracarbonsäuredianhydrid und 0,208 g (= 0,2 Gew.-%) p-Toluolsulfonsäure versetzt und anschließend weiter erwärmt. Im Verlauf der Reaktionszeit steigt die Temperatur auf ca. 115 °C. Nach 4,5 Stunden wird die Säurezahl mit 24,87 mg KOH/g bestimmt, das farblose Produkt abgelassen und abgekühlt.

Die Anwendung der erfindungsgemäßen Verdickungsmittel ist in den folgenden Beispielen näher beschrieben. Dabei wurden Tensidlösungen mit einem Gehalt von 10 % Aktivsubstanz verdickt. Im einzelnen handelt es sich dabei um

MARLINAT® CM 105 - Fettalkoholpolyglykolethercarbonsäure-Na-Salz

MARLINAT® 24/70 - Fettalkoholethersulfat

MARLIPAL® 24/7 - Fettalkoholpolyglykolether

MARLON® PS 65 - sek. Paraffinsulfonat

Die Viskositäten wurden mit einem rechnergesteuerten Rotationsviskosimeter (Typ: Haake RV 12) bestimmt: $D = 2 \, \text{sec}^{-1}$, $T = 25$ °C.

### Anwendungsbeispiel 1

Verdickungsmittel: Umsetzungsprodukt aus Pyromellithsäuredianhydrid und $C_{16-18}$-Fettalkoholpolyglykolether (100 mol Ethylenoxid) im Molverhältnis 1 : 2 gemäß Herstellbeispiel 1.

| Gehalt an Verdickungsmittel [%] | Tensid | |
|---|---|---|
| | MARLINAT® CM 105 | MARLIPAL® 24/7 |
| | Viskosität [mPas] | |
| 0 | < 5 | < 5 |
| 3 | 32 | 1 940 |
| 6 | 7 115 | 8 350 |
| 10 | 54 900 | 34 700 |

Anwendungsbeispiel 2

Verdickungsmittel: Umsetzungsprodukt aus Butan-1,2,3,4-tetracarbonsäuredianhydrid mit $C_{16-18}$-Fettalkoholpolyglykolether (50 mol EO) im Molverhältnis 1 : 2 gemäß Herstellbeispiel 2.

| Gehalt an Verdickungsmittel [%] | Tensid |
|---|---|
| | MARLON® PS 65 |
| | Viskosität [mPas] |
| 0 | < 5 |
| 3 | 592 |
| 6 | 13 900 |
| 10 | 64 500 |

Anwendungsbeispiel 3

Verdickungsmittel: Umsetzungsprodukt aus Pyromellithsäuredianhydrid mit Olelyalkoholpolyglykolether (100 mol EO) im Molverhältnis 1 : 2 analog Herstellbeispiel 1.

| Gehalt an Verdickungsmittel [%] | Tensid | | |
|---|---|---|---|
| | MARLINAT® CM 105 | MARLINAT® 24/70 | MARLIPAL® 24/7 |
| | Viskosität [mPas] | | |
| 0 | < 5 | < 5 | < 5 |
| 3 | 39 | 13 | 1 340 |
| 6 | 1 830 | 2 210 | 6 710 |
| 10 | 20 000 | 9 930 | 18 500 |

Anwendungsbeispiel 4

Verdickungsmittel: Umsetzungsprodukt aus Pyromellithsäuredianhydrid mit Oleylalkoholpolyglykolether (40 mol EO) im Molverhältnis 1 : 2 analog Herstellbeispiel 1.

| Gehalt an Verdickungsmittel [%] | Tensid | | |
|---|---|---|---|
| | MARLINAT® CM 105 | MARLIPAL® 24/7 | MARLON® PS 65 |
| | Viskosität [mPas] | | |
| 0 | < 5 | < 5 | < 5 |
| 3 | 915 | 2 370 | 24 |
| 6 | 29 950 | 15 650 | 1 850 |
| 10 | 70 300 | 53 900 | 12 200 |

Anwendungsbeispiel 5

Verdickungsmittel: Umsetzungsprodukt aus Pyromellithsäuredianhydrid mit $C_{16-18}$ Fettalkoholpolyglykolether (25 mol EO) im Molverhältnis 1 : 2 analog Herstellbeispiel 1.

| Gehalt an Verdickungsmittel [%] | Tensid | | |
|---|---|---|---|
| | MARLINAT® 24/70 | MARLIPAL® 24/7 | MARLON® PS 65 |
| | Viskosität [mPas] | | |
| 0 | < 5 | < 5 | < 5 |
| 3 | 5 | 345 | 9 |
| 6 | 2 810 | 5 330 | 1 310 |
| 10 | 10 900 | 35 900 | 40 000 |

Anwendungsbeispiel 6 (Vergleichsbeispiel A)

Verdickungsmittel: Natriumchlorid

5

| Gehalt an Verdickungsmittel [%] | Tensid | | | |
|---|---|---|---|---|
| | MARLINAT® CM 105 | MARLINAT® 24/70 | MARLIPAL® 24/7 | MARLON® PS 65 |
| | Viskosität [mPas] | | | |
| 0 | < 5 | < 5 | < 5 | < 5 |
| 3 | < 5 | 26 | 14 | nicht lösl. |
| 6 | < 5 | 13 800 | 176 | nicht lösl. |
| 10 | < 5 | 1 323 | 89 | nicht lösl. |

Anwendungsbeispiel 7 (Vergleichsbeispiel B)

Verdickungsmittel: Kokosfettsäurediethanolamid

| Gehalt an Verdikungsmittel [%] | Tensid | | | |
|---|---|---|---|---|
| | MARLINAT® CM 105 | MARLINAT® 24/70 | MARLIPAL® 24/7 | MARLON® PS 65 |
| | Viskosität [mPas] | | | |
| 0 | < 5 | < 5 | < 5 | < 5 |
| 3 | 5 | < 5 | < 5 | < 5 |
| 6 | < 5 | < 5 | 135 | 11 |
| 10 | 17 | 312 | 145 | 122 |

## Ansprüche

1. Verwendung von Umsetzungsprodukten von Polyanhydriden, die mindestens zwei Anhydridfunktionen pro Molekül aufweisen, mit Polyglykolethern der Formel R-O(CHR$_1$-CHR$_2$-O)$_x$ H als Verdickungsmittel für wäßrige Tensidformulierungen, wobei R für gesättigte oder ungesättigte Alkylreste mit 8 bis 24 C-Atomen oder für Alkylarylreste, R$_1$ und R$_2$ für H, CH$_3$, C$_2$H$_5$ mit der Maßgabe, daß mindestens ein Rest H ist, und x für ganze Zahlen von 20 bis 150 stehen und daß das Verhältnis von Polyglykolether zu Anhydridfunktion 0,5 : 1 bis 2 : 1 ist.

2. Verwendung von Umsetzungsprodukten nach Anspruch 1,
dadurch gekennzeichnet,
daß R für einen gesättigten oder ungesättigten Alkylrest mit 14 bis 20 C-Atomen steht.

3. Verwendung von Umsetzungsprodukten nach Anspruch 1,
dadurch gekennzeichnet,
daß x für ganze Zahlen von 30 bis 100 steht.

4. Verwendung von Umsetzungsprodukten nach Anspruch 1,
dadurch gekennzeichnet,
daß das Verhältnis von Polyglykolether zu Anhydridfunktion 0,8 : 1 bis 1,3 : 1 ist.

5. Verwendung von Umsetzungsprodukten nach Anspruch 1,
dadurch gekennzeichnet,
daß die Einsatzkonzentration 0,1 bis 10 % Verdickungsmittel, bezogen auf wäßrige Tensidformulierung, beträgt.

6. Verwendung von Umsetzungsprodukten nach Anspruch 9,

dadurch gekennzeichnet,

daß die Einsatzkonzentration 1 bis 5 % Verdickungsmittel, bezogen auf wäßrige Tensidformulierung, beträgt.

7. Verfahren zur Herstellung der Umsetzungsprodukte nach Anspruch 1,

dadurch gekennzeichnet,

daß die beiden Komponenten mit oder ohne Veresterungskatalysatoren bei milden Bedingungen umgesetzt werden.

8. Verfahren nach Anspruch 2,

dadurch gekennzeichnet,

daß als Veresterungskatalysator p-Toluolsulfonsäure oder 1-Methylimidazol verwendet werden.

9. Verfahren nach Anspruch 2 und 3,

dadurch gekennzeichnet,

daß die Umsetzung unter Normaldruck und bei 80 bis 140 °C durchgeführt wird.

10. Verfahren nach Anspruch 2 bis 4,

dadurch gekennzeichnet,

daß die Umsetzung bei 100 bis 120 °C durchgeführt wird.